Europäisches Patentamt

European Patent Office

Office européen des brevets

(11) Veröffentlichungsnummer: **0 209 798 B1**

# EUROPÄISCHE PATENTSCHRIFT

(45) Veröffentlichungstag der Patentschrift: **03.06.92**

(21) Anmeldenummer: **86109414.2**

(22) Anmeldetag: **10.07.86**

(51) Int. Cl.⁵: **C07C 47/565**, C07C 45/80, C07C 45/82, C07C 45/81, C07C 45/36, C07C 45/79

(54) **Verfahren zur Isolierung von p-Hydroxybenzaldehyd.**

(30) Priorität: **19.07.85 DE 3525848**

(43) Veröffentlichungstag der Anmeldung:
**28.01.87 Patentblatt 87/05**

(45) Bekanntmachung des Hinweises auf die Patenterteilung:
**03.06.92 Patentblatt 92/23**

(84) Benannte Vertragsstaaten:
**BE CH DE FR GB IT LI NL**

(56) Entgegenhaltungen:
**EP-A- 0 012 939**
**FR-A- 2 379 500**
**US-A- 4 453 016**

(73) Patentinhaber: **HOECHST AKTIENGESELL-SCHAFT**
**Postfach 80 03 20**
**W-6230 Frankfurt am Main 80(DE)**

(72) Erfinder: **Röhrscheid, Freimund, Dr.**
**Amselweg 24**
**W-6233 Kelkheim(DE)**

## Beschreibung

Die Erfindung betrifft die Isolierung von p-Hydroxybenzaldehyd aus dem durch Oxidation von p-Kresol mit Sauerstoff erhaltenen Reaktionsgemisch. p-Hydroxybenzaldehyd und seine Derivate finden Verwendung als Zwischenprodukte in der Herstellung von Farbstoffen, Pharmazeutika, Pflanzenschutzmitteln und Polymerharzen.

In der EP-PS 0 012 939, der US-PS 4 453 016 und der US-PS 4 471 140 werden Verfahren beschrieben, nach denen p-Kresol in Gegenwart von Na- oder K-Hydroxid und einer Co-, Mn-, Cr-, oder Ni-Verbindung mit molekularem Sauerstoff zum Alkalisalz des p-Hydroxybenzaldehyds oxidiert wird.

Eine besonders günstige Ausführungsform ist die Verwendung von Methanol als Lösemittel und eines Co-Salzes als Katalysator. Nach Beendigung der Reaktion liegt das Natrium- oder Kaliumsalz des p-Hydroxybenzaldehyds, teilweise als Suspension, zusammen mit Co-, Ni-, Cr- oder Mn-Oxidhydratschlamm und gelöstem Alkalihydroxid in Methanol vor. Für die Aufgabe, den p-Hydroxbenzaldehyd aus dieser Mischung quantitativ in reiner Form zu isolieren, wird in den oben genannten Veröffentlichungen keine technisch verwertbare Lösung angeboten.

In der EP-PS 0 012 939, S. 4, Zeile 40 wird für die Isolierung des Produktes vorgeschlagen, die Reaktionsmischung durch Abdampfen des Methanols zu konzentrieren, das Konzentrat mit Wasser zu versetzen und anzusäuern und mit einem organischen Lösemittel den gewünschten Aldehyd zu extrahieren. Diese Verfahrensweise ist jedoch mit erheblichen Schwierigkeiten verbunden:

a) beim Abdampfen des Methanols scheiden sich an der Reaktorwand feste Krusten ab, die den Rührer blockieren und den Wärmefluß behindern. Dadurch ist die Rückgewinnung des Methanols nur unvollständig möglich und die gesteinsartigen Krusten sind nur schwer in Wasser aufzulösen.

b) Beim Ansäuern des mit Wasser versetzten Konzentrats fällt der p-Hydroxybenzaldehyd als braune klebrige Masse an, die Nebenprodukte und nicht umgesetztes p-Kresol enthält. Da der p-Hydroxybenzaldehyd sich auch im Vakuum nicht unzersetzt destillieren läßt, muß er mühsam durch Umkristallisieren gereinigt werden, im Beispiel 1 der US-PS 4 471 140 aus einer Mischung von Chloroform/Hexan.

c) Bei der Freisetzung des p-Hydroxybenzaldehyds durch Säure geht der Metallkatalysator als Salz in Lösung und muß anschließend aus dem Abwasser entfernt werden.

Es wurden nun Verfahren gefunden, die die oben beschriebenen Probleme vermeiden und durch die der gebildete Hydroxybenzaldehyd fast quantitativ in hoher Reiheit isoliert werden kann.

Ein Gegenstand der Erfindung ist ein Verfahren zur Isolierung von p-Hydroxybenzaldehyd aus dem durch Oxidation von p-Kresol mit Sauerstoff oder sauerstoffhaltigen Gasen in Methanol in Gegenwart von Na- oder K-Hydroxid und eines Mn-, Ni-, Cr- oder Co-Salzes erhaltenen Reaktionsgemisch, dadurch gekennzeichnet, daß man dem Reaktionsgemisch Wasser zufügt, die entstehende Lösung erwärmt und das ausgefallene Mn-, Ni-, Cr- oder Co-Oxidhydrat abfiltriert, aus dem Filtrat das Methanol abdestilliert, die zurückbleibende wäßrige Lösung abkühlt und so den p-Hydroxybenzaldehyd als Na- oder K-Salz auskristallisieren läßt.

Der Reaktionsmischung werden dabei im allgemeinen 2 bis 6, vorzugsweise 2,5 bis 4 Gewichtsteile Wasser pro 1 Gewichtsteil bei der Oxidation eingesetztes p-Kresol zugefügt und die entstehende Lösung auf etwa 50-90 °C erwärmt. Dabei entsteht eine Lösung, in der die Na- und K-Salze des p-Hydroxybenzaldehyds überraschenderweise besonders gut löslich sind. Diese Lösung wird heiß filtriert, wird heiß filtriert, um das ausgefallene Oxidhydrat abzutrennen. Letzteres gelangt dadurch nicht ins Abwasser und kann als Katalysator wieder eingesetzt werden. Vom orangefarbenen Filtrat wird jetzt in einer Destillationskolonne das Methanol abdestilliert. Da Methanol mit Wasser kein Azeotrop bildet, kann so das Methanol in wasserfreier Form abgetrennt und für eine erneute Umsetzung verwendet werden. Die wäßrige Lösung aus dem Sumpf der Kolonne wird unter Rühren abgekühlt, vorzugsweise auf ca. 5°. Das Natriumsalz des p-Hydroxybenzaldehyds mit der Formel Na-$(O \cdot C_6H_4 \cdot CHO) \cdot 2H_2O$ kristallisiert in Form von Blättchen aus, während das Kaliumsalz mit einem Hydratwasser kristallisiert. Beide lassen sich gut abfiltrieren.

Durch die im Beispiel beschriebene Aufarbeitung der Mutterlauge wird gezeigt, daß 98 % des entstandenen p-Hydroxybenzaldehyds nach der beschriebenen Verfahrensweise als Natriumsalz ausgefällt wurden.

Ein weiterer Gegenstand der Erfindung ist ein Verfahren zur Isolierung von p-Hydroxybenzaldehyd aus dem durch Oxidation von p-Kresol mit Sauerstoff oder sauerstoffhaltigen Gasen in Methanol in Gegenwart von Na- oder K-Hydroxid und eines Mn-, Ni-, Cr- oder Co-Salzes erhaltenen Reaktionsgemisch, dadurch gekennzeichnet, daß man das Reaktionsgemisch durch Versprühen trocknet, die löslichen Bestandteile der Trockensubstanz mit heißem Wasser löst, das dabei ungelöste Mn-, Ni-, Cr- oder Co-Oxidhydrat abfiltriert und durch Abkühlen des Filtrats den p-Hydroxybenzaldehyd als Na- oder K-Salz auskristallisieren läßt.

Die Wassermenge und deren Temperatur sowie die Temperatur beim Auskristallisieren ist wieder wie bei der zuvor beschriebenen Verfahrensvariante.

Ein weiterer Gegenstand der Erfindung ist ein Verfahren zur Isolierung von p-Hydroxybenzaldehyd aus dem durch Oxidation von p-Kresol mit Sauerstoff oder sarnerstoffhaltigen Gasen in Methanol in Gegenwart von Na- oder K-Hydroxid und eines Mn-, Ni-, Cr- oder Co-Salzes erhaltenen Reaktionsgemisch, dadurch gekennzeichnet, daß man das Reaktionsgemisch mit Methanol verdünnt, das ungelöste Mn-, Ni-, Cr- oder Co-Oxidhydrat abfiltriert, das Filtrat durch Versprühen trocknet, die entstandene Trockensubstanz mit heißem Wasser löst und durch Abkühlen der Lösung den p-Hydroxybenzaldehyd als Na- oder K-Salz auskristallisieren läßt.

Die zur Verdünnung eingesetzte Methanolmenge beträgt vorzugsweise etwa 0,2 bis 1 Teil Methanol pro 1 Teil Reaktionsgemisch. Die Wassermenge und deren Temperatur sowie die Temperatur beim Auskristallisieren ist wieder wie bei den beiden zuvor beschriebenen Verfahrensvarianten.

Die Löslichkeit des Na- oder K-Salzes des p-Hydroxybenzaldehyds wird durch Zugabe der betreffenden Alkaliionen, z.B. in Form der Hydroxide oder Halogenide stark verringert. Daher kann z.B. das abfiltrierte Kristallisat des Natriumsalzes ohne merkliche Verluste mit einer Kochsalzlösung gewaschen werden.

Das erfindungsgemäß isolierte Natrium- oder Kaliumsalz des p-Hydroxybenzaldehyds ist eine für Folgereaktionen besonders günstige Form des P-Hydroxybenzaldehyds; das Salz kann z.B. direkt für die Umsetzung mit Dimethylsulfat zum Anisaldehyd eingesetzt werden.

Wenn gewünscht, läßt sich aber aus dem Alkalisalz durch Zugabe von Säuren leicht der P-Hydroxybenzaldehyd freisetzen. Seine Reinheit beträgt über 99,5 %.

Vorzugsweise oxidiert man das p-Kresol in Gegenwart von Natriumhydroxid, da das dann entstehende Natriumsalz des p-Hydroxybenzaldehyds besonders wenig löslich in kaltem Wasser ist.

Beispiel

Oxidationsreaktion*:

In einem 4 l-Kolben mit Gaseinleitrohr, Thermometer, Rückflußkühler und Impeller-Rührer wurde eine Lösung aus
432,6 g (4 Mol) p-Kresol
480 g (12 Mol) Natriumhydroxid, fest
9,52 g (0,04 Mol) CoCl$_2 \cdot$6H$_2$O und
1000 g (1,26 l) Methanol
bei 60° und 800 Umdrehungen/Min mit Sauerstoff unter Normaldruck oxidiert. Nach 11 Stunden wurde die Oxidation abgebrochen; der O$_2$-Verbrauch war ca. 90 l, entsprechend 100 % des theoretischen Bedarfs. Der Umsetzungsgrad des p-Kresols war 93,1 %.

Aufarbeitung:

Die oxidierte Lösung wurde mit 1200 g Wasser vermischt und auf 70° erwärmt. Dann wurde zur Abtrennung des ausgefallenen Kobaltoxidhydrats bei 50-60° über ein beheiztes Filter Filter filtriert. Von der orangefarbenen Lösung wurde an einer Kolonne (1,20 m, Glasringe 4 x 4 mm) das Methanol (99:9 %ig) abdestilliert. Das Methanol ging bei 35,5° C/260 mbar über, die Temperatur des Sumpfes stieg von 53 auf 73 ° C.

Die nach dem Abdestillieren des Methanols zurückbleibende wäßrige Lösung wurde auf 5° abgekühlt, wobei das Natriumsalz des p-Hydroxybenzaldehyds in Form von Blättchen auskristallisierte. Die Kristalle wurden scharf abgesaugt und mit etwas Natronlauge gewaschen. Das fast farblose Natriumsalz des p-Hydroxybenzaldehyds wurde getrocknet.

Um den p-Hydroxybenzaldehyd freizusetzen, wurde das Natriumsalz in 1000 g Wasser auf 60° erwärmt und dann mit 200 ml konz. Salzsäure umgesetzt. Der farblose p-Hydroxybenzaldehyd wurde nach dem Abkühlen abgesaugt und dreimal mit 100 ml Wasser gewaschen und bei 40 ° C/33 mbar getrocknet.
Ausbeute 289,8 g (= 59,4 % d.Th., Selektivität 63,8 %)
Fp. 116°, Reinheit 99,8 %.

Aufarbeitung der Mutterlauge:

Die alkalische Mutterlauge, die nach der Abtrennung des Natriumsalzes des p-Hydroxybenzaldehyds anfiel, wurde auf pH 3 angesäuert. Es schied sich eine ölige Substanz ab, die mit 3 x 250 ml Ethylacetat extrahiert wurde. Das Ethylacetat wurde über eine Kolonne abdestilliert, der Rückstand wurde gaschromatographisch auf seine Zusammensetzung analysiert; er enthielt:
29,8 g p-Kresol (6,9 %)
5,2 g p-Hydroxybenzaldehyd (1,1 % d.Th.)
24,6 g p-Hydroxybenzylmethylether (4,5 % d.Th.)
Die Aufarbeitung der Mutterlauge zeigt, daß die erfindungsgemäße Abtrennung des Natriumsalzes von p-Hydroxybenzaldehyd fast quantitativ gelingt.

**Patentansprüche**

* Die Oxidationsreaktion wurde gemäß Beispiel 1 der EP-PS 0 012 939, aber im größeren Maßstab ausgeführt.

1. Verfahren zur Isolierung von p-Hydroxybenzaldehyd aus dem durch Oxidation von p-Kresol mit Sauerstoff oder sauerstoffhaltigen Gasen in Methanol in Gegenwart von Na- oder K-Hydroxid und eines Mn-, Ni-, Cr- oder Co-Salzes erhaltenen Reaktionsgemisch, dadurch gekennzeichnet, daß man den Reaktionsgemisch Wasser zufügt, die entstehende Lösung erwärmt und das ausgefallene Mn-, Ni-, Cr- oder Co-Oxidhydrat abfiltriert, aus dem Filtrat das Methanol abdestilliert, die zurückbleibende wäßrige Lösung abkühlt und so den p-Hydroxybenzaldehyd als Na- oder K-Salz auskristallisieren läßt.

2. Verfahren nach Anspruch 1, dadurch gekennzeichnet, daß man dem Reaktionsgemisch 2 bis 6 Gewichtsteile Wasser pro 1 Gewichtsteil bei der Oxidation eingesetztem p-Kresol zufügt.

3. Verfahren nach Anspruch 1 oder 2, dadurch gekennzeichnet, daß man die durch Zufügen von Wasser entstehende Lösung auf 50-90 °C erwärmt.

4. Verfahren zur Isolierung von p-Hydroxybenzaldehyd aus dem durch Oxidation von p-Kresol mit Sauerstoff oder sauerstoffhaltigen Gasen in Methanol in Gegenwart von Na- oder K-Hydroxid und eines Mn-, Ni-, Cr- oder Co-Salzes erhaltenen Reaktionsgemisch, dadurch gekennzeichent, daß man das Reaktionsgemisch durch Versprühen trocknet, die löslichen Bestandteile der Trockensubstanz mit heißem Wasser löst, das dabei ungelöste Mn-, Ni-, Cr- oder Co-Oxidhydrat abfiltriert und durch Abkühlen des Filtrats den p-Hydroxybenzaldehyd als Na- oder K-Salz auskristallisieren läßt.

5. Verfahren nach Anspruch 4, dadurch gekennzeichnet, daß man die löslichen Bestandteile der Trockensubstanz mit 2 bis 6 Gewichtsteilen heißem Wasser pro 1 Gewichtsteil bei der Oxidation eingesetztem p-Kresol löst.

6. Verfahren nach Anspruch 4 oder 5, dadurch gekennzeichnet, daß das heiße Wasser eine Temperatur von 50-90 °C hat.

7. Verfahren zur Isolierung von P-Hydroxybenzaldehyd aus dem durch Oxidation von p-Kresol mit Sauerstoff oder sauerstoffhaltigen Gasen in Methanol in Gegenwart von Na- oder K-Hydroxid und eines Mn-, Ni-, Cr- oder Co-Salzes erhaltenen Reaktionsgemisch, dadurch gekennzeichnet, daß man das Reaktionsgemisch mit Methanol verdünnt, das ungelöste Mn-, Ni-, Cr-, oder Co-Oxidhydrat abfiltriert, das Filtrat durch Versprühen trocknet, die entstandene Trockensubstanz mit heißem Wasser löst und durch Abkühlen der Lösung den p-Hydroxybenzaldehyd als Na- oder K-Salz auskristallisieren läßt.

8. Verfahren nach Anspruch 7, dadurch gekennzeichnet, daß man das Reaktionsgemisch mit 0,2 bis 1 Teil Methanol pro 1 Teil Reaktionsgemisch verdünnt.

9. Verfahren nach Anspruch 7 oder 8, dadurch gekennzeichnet, daß man die Trockensubstanz mit 2 bis 6 Gewichtsteilen heißem Wasser pro 1 Gewichtsteil bei der Oxidation eingesetztem p-Kresol löst.

10. Verfahren nach einem der Ansprüche 7 bis 9, dadurch gekennzeichnet, daß das heiße Wasser eine Temperatur von 50-90 °C hat.

**Claims**

1. A process for the isolation of p-hydroxybenzaldehyde from the reaction mixture obtained by oxidizing p-cresol with oxygen or oxygen-containing gases in methanol in the presence of Na or K hydroxide and an Mn, Ni, Cr or Co salt, which comprises adding water to the reaction mixture, heating the resulting solution and filtering off the precipitated Mn, Ni, Cr or Co oxide-hydrate, removing the methanol from the filtrate by distillation, cooling the residual aqueous solution and thus allowing the p-hydroxybenzaldehyde to crystallize out in the from of the Na or K salt.

2. The process as claimed in claim 1, wherein 2 to 6 parts by weight of water per part by weight of p-cresol employed in the oxidation is added to the reaction mixture.

3. The process as claimed in claim 1 or 2, wherein the solution formed by adding water is heated to 50 - 90 °C.

4. A process for the isolation of p-hydroxybenzaldehyde from the reaction mixture obtained by oxidizing p-cresol with oxygen or oxygen-containing gases in methanol in the presence of Na or K hydroxide and an Mn, Ni, Cr or Co salt, which comprises drying the reaction mixture by atomization, dissolving the soluble constituents of the dry substance in hot water, filtering off the Mn, Ni, Cr or Co oxide-hydrate, which is undissolved thereby, and allowing the p-hydroxybenzaldehyde rto crystallize out as the Na or K salt by cooling the filtrate.

5. The process as claimed in claim 4, wherein the soluble constituents of the dry substance are dissolved in 2 to 6 parts by weight of hot water per part by weight of p-cresol employed in the oxidation.

6. The process as claimed in claim 4 or 5, wherein the hot water is at a temperature of 50 - 90°C.

7. A process for the isolation of p-hydroxybenzaldehyde from the reaction mixture obtained by oxidizing p-cresol with oxygen or oxygen-containing gases in methanol in the presence of Na or K hydroxide and an Mn, Ni. Cr or Co salt, which comprises diluting the reaction mixture with methanol, filtering off the undissolved Mn, Ni, Cr or Co oxide-hydrate, drying the filtrate by atomization, dissolving the dry substance formed in hot water, and allowing the p-hydroxybenzaldehyde to crystallize out as the Na or K salt by cooling the solution.

8. the process as claimed in claim 7, wherein the reaction mixture is diluted with 0.2 to 1 part of methanol per part of reaction mixture.

9. the process as claimed in claim 7 or 8, wherein the dry substance is dissolved in 2 to 6 parts by weight of hot water per part by weight of p-cresol employed in the oxidation.

10. The process as claimed in one of claims 7 to 9, wherein the hot water is at a temperature of 50 - 90°C.

**Revendications**

1. Procédé pour isoler le p-hydroxy-benzaldéhyde à partir du mélange réactionnel obtenu par oxydation du p-crésol par de l'oxygène ou des gaz contenant de l'oxygène, dans du méthanol, en présence d'hydroxyde de sodium ou de potassium et d'un sel de Mn, de Ni, de Cr ou de Co, procédé caractérisé en ce qu'on ajoute de l'eau au mélange réactionnel, on chauffe la solution formée, on sépare par filtration l'oxyde hydraté de Mn, Ni, Cr ou Co qui a précipité, on chasse le méthanol du filtrat par distillation, on refroidit la solution aqueuse restante et on fait ainsi cristalliser le p-hydroxy-benzaldéhyde sous la forme de son sel sodique ou potassique.

2. Procédé selon la revendication 1 caractérisé en ce qu'on ajoute au mélange réactionnel de 2 à 6 parties en poids d'eau pour 1 partie en poids du p-crésol mis en jeu lors de l'oxydation;

3. Procédé selon l'une des revendications 1 et 2, caractérisé en ce qu'on chauffe à 50-90°C la solution qui s'est formée lors de l'addition d'eau.

4. Procédé pour isoler le p-hydroxy-benzaldéhyde à partir du mélange réactionnel obtenu par oxydation du p-crésol au moyen d'oxygène ou de gaz contenant de l'oxygène, dans du méthanol, en présence d'hydroxyde de Na ou de K et d'un sel de Mn, Ni, Cr ou Co, procédé caractérisé en ce qu'on sèche le mélange réactionnel par pulvérisation, on dissout dans de l'eau chaude les constituants de la matière sèche qui sont solubles, on sépare par filtration l'hydrate d'oxyde de Mn, Ni, Cr ou Co qui est alors resté à l'état non dissous, et, en refroidissant le filtrat, on fait cristalliser le p-hydroxy-benzaldéhyde sous la forme de son sel sodique ou potassique.

5. Procédé selon la revendication 4 caractérisé en ce qu'on dissout les constituants solubles de la substance sèche avec de 2 à 6 parties en poids d'eau chaude pour 1 partie en poids du p-crésol mis en jeu lors de l'oxydation.

6. Procédé selon l'une des revendications 4 et 5, caractérisé en ce que l'eau chaude est à une température de 50 à 90°C.

7. Procédé pour isoler le p-hydroxy-benzaldéhyde à partir du mélange réactionnel obtenu par oxydation du p-crésol au moyen d'oxygène ou de gaz contenant de l'oxygène, dans du méthanol, en présence d'hydroxyde de Na ou de K et d'un sel de Mn, Ni, Cr ou Co, procédé caractérisé en ce qu'on dilue le mélange réactionnel avec du méthanol, on sépare par filtration l'oxyde hydraté de Mn, Ni, Cr ou Co non dissous, on sèche le filtrat par pulvérisation, on dissout avec de l'eau chaude la substance sèche formée et, en refroidissant la solution, on fait cristalliser le p-hydroxy-benzaldéhyde sous la forme de son sel sodique ou potassique.

8. Procédé selon la revendication 7 caractérisé en ce qu'on dilue le mélange réactionnel avec de 0,2 à 1 partie de méthanol pour 1 partie du mélange réactionnel.

9. Procédé selon l'une des revendications 7 et 8, caractérisé en ce qu'on dissout la substance sèche avec de 2 à 6 parties en poids d'eau chaude pour 1 partie en poids du p-crésol mis en jeu lors de l'oxydation.

10. Procédé selon l'une quelconque des revendications 7 à 9, caractérisé en ce que l'eau chaude est à une température de 50 à 90°C.